# NEW EUROPEAN PATENT SPECIFICATION

(11) **EP 0 123 465 B2**
(45) Date of publication and mention of the opposition decision: **06.11.1996**
(45) Mention of the grant of the patent: 30.10.1991
(21) Application number: 84302322.7
(22) Date of filing: 05.04.1984
(51) Int. Cl.: A61L 15/16

(54) **Surgical adhesive dressing**
Chirurgisches Heftpflaster
Pansement chirurgical adhésif

(30) Priority: 13.04.1983 GB 8309993
(43) Date of publication of application: 31.10.1984
(73) Proprietor: SMITH & NEPHEW plc, London WC2R 3BP (GB)
(72) Inventor: Rawlings, David Alan, Stansted Mountfitchet Essex (GB); Potter, William Duncan, Bishops Stortford Hertfordshire (GB)
(74) Representative: W.P. Thompson & Co.

(56) References cited:
- EP-A- 0 028 452
- EP-A- 0 072 258
- EP-A- 0 091 800
- GB-A- 648 733
- GB-A- 1 280 631
- GB-A- 2 093 190
- US-A- 3 975 350

## Description

The present invention relates to adhesive dressings for use on the human body. More particularly this invention relates to adhesive surgical dressings suitable for use on both exuding wounds and non-exuding wounds.

Moisture vapour permeable thin films coated with adhesive were disclosed in British Patent No. 1,280.631 and U.S. Patent No. 3,645,835 as being suitable for use as surgical dressings. In recent years one such film has come to prominence under the trade mark "OpSite" and has found use as a surgical dressing, for example for covering burns, donor sites, surgical incisions, intravenous catheter sites and the like. The known dressings in commercial use have proved useful because they keep out bacteria owing to the microscopically continuous nature of the film and adhesive layer but do not cause maceration of the skin to which it is applied because both the film and the adhesives layer have high moisture vapour permeability (MVP). One problem with presently available high MVP dressings (MVP) generally between 300 and 800g/m² and independent of the nature of the wound, for example the MVP of OpSite is approximately 830gm⁻²24hr⁻¹) is that the MVP is not high enough for some uses such as covering exuding wounds when an unsightly blister can occur. However it has not been thought practible simply to increase the MVP of the product overall since this would lead to drying out some wounds with a consequent reduction in the rate of healing. It has now been discovered that it is possible to alleviate the known disadvantages of conventional surgical dressings by providing dressings which transmit more moisture vapour when in contact with a wetter wound than they do when in contact with a dryer wound.

Accordingly the present invention provides a surgical dressing which consists essentially of a film which carries over its face an adhesive layer for securing the dressing to the body characterised in that (a) the film is continuous, hydrophilic and elastomeric and consists essentially of polyether polyurethane, polyether polyamide block copolymer or polyether polyester block copolymer and in contact with water has a higher MVP than when in contact with moisture vapour but not water (b) the adhesive layer is porous, allows access of water to the film when the water is in contact with the adhesive layer and is applied over the whole of the operative area of the surgical dressing so that (c) said surgical dressing has a wet MVP of not less than 2500g/m² when the adhesive layer is in contact with water and has a dry MVP of not more than 2000g/m² when the adhesive is in contact with moisture vapour but not water, whereby the dressing is suitable for use on exuding wounds and on non-exuding wounds, the pores in said adhesive being from 20 to 300µm in diameter and accounting for 10 to 50% of the area of the adhesive.

When used herein with reference to " contact" the term "water" means liquid water (as opposed to moisture vapour) unless otherwise specified. When used herein MVP units are g/m²/24hrs/37°C/100-10% relative humidity and are generally abbreviated to g/m².

Suitable test methods for determining the MVP of a dressing or its components are set forth in the Demonstration 1 hereinafter. When MVP values quoted thereinafter are referred to as "wet-MVP" they refer to values obtained with the adhesive face in contact with water and when referred to as "dry-MVP" they refer to values obtained with the adhesive face not in contact with water (but in contact with moist air).

More suitable the dressing of this invention will have a wet-MVP of not less than 3000g/m², most suitably will have a wet-MVP of not less than 3200g/m² and preferably will have a wet-MVP of not less than 5000g/m².

More suitably the dressing of this invention will have a dry-MVP of not more than 1500g/m², most suitably will have a dry-MVP of not more than 1400g/m² and preferably will have a dry-MVP of not more than 1200g/m².

The film used in this invention may comprise any polyether polyurethane, polyether polyamide block copolymer or polyether polyester block copolymer which has a sufficiently higher wet-MVP than dry-MVP to produce the desired MVP parameters in the dressing. The method set out in the Demonstration 1 may be employed to determine whether the film material exhibits the desired MVP when in contact with water. An elastomeric film conforms to the movement of the skin when the dressing is in use.

The film material used in this invention will by hydrophilic, that is, will absorb water when immersed therein. The film material when hydrated will contain 5% to 50% water (w/w at 20°C), more aptly from 10% to 40% of water and favourably from 20% to 30% of water. The film will not be soluble in water.

The film employed will be a continuous film that is will be free of holes (whether microporous or macroporous) which allow the passage of bacteria.

The desirable properties of this invention may be best obtained by employing a film of hydrophilic polyether polyurethane in combination with an adhesive layer adapted to allow access of water to the film when water is presented to the adhesive face of the dressing.

Most suitably the film will be from 15 to 80µm thick, will more usually be from 20 to 60µm thick and will preferably be from 25 to 50µm thick, for example 30, 35 or 40 µm thick.

Aptly the film will be formed from a hydrophilic polyether polyurethane which when hydrated contains from 5% to 50% of water more aptly from 10% to 40% of water and favourably from 20% to 30% water.

In order to enable visual observation of the wound it is desirably for the film used in this invention to be transparent. This in turn requires that the film should be capable of being self supporting, that is sufficiently coherent when wet or dry to be used without recourse to additional support such as a fabric, for example a gauze, net or the like (which in any event would render them likely to bacterial penetration). It has been found that polyether polyurethanes are particularly suitable for use in the formation of such films. Favoured polyether polyurethanes are essentially free of reactive substituents such as hydroxyl or carboxy groups. It has been found that certain apt polyurethanes for use in this invention are random polymers containing units derived from diolic compounds and di-isocyanates and which use either diols, for example ethylene glycol, or diamines, for example ethylene diamine, as chain extenders.

Suitable polyurethanes are disclosed in British Patent Specification No. 2093190A at page 3 lines 16 to 74.

The adhesive layer present on the body contacting surface of the film is a porous layer which allows access of liquid water to the film. Most suitably the pores will be large in comparison to the thickness of the adhesive layer. Favourably the diameters of the pores are 2 to 3 times the thickness of the adhesive layer. The scanning electron microscope may be used to examine the porous adhesive layer. The pores will be from about 20 to 300µm in diameter. The pores account from 10-50% of the area of the adhesive and preferably 15-40% of the area of the adhesive and most preferably 20-30% of the area of the adhesive. The use of a porous layer in such a manner has been found to be highly beneficial in allowing the desirable variability of the MVP to be achieved.

The size of the pores and area of adhesive accounted for by the pores may be measured using a scanning electron microscope (SEM). The release paper or protector is removed from a dressing of the invention and the surface of the adhesive layer is photographed using the SEM. The magnification used is such as to allow a large area of the surface of the adhesive to be photographed while still permitting accurate measurement of the dimensions of the pores. The % area accounted from by the pores may be calculated manually be measuring the area of the pores per unit area or automatically by using an image analyser. The distribution of pore sizes can similarly be calculated. (Alternatively the pores in the adhesive may be photographed after spreading and drying on a release paper but prior to transfer coating under mild conditions; the transfer coating does not greatly effect the physical characteristics of the adhesive layer).

The adhesive is generally employed at a mass per unit area of 20 to 80g/m², more aptly 20 to 45g/m² and favourably from 25 to 35g/m².

The adhesive is applied over the whole operative area of the dressing (this may conveniently be over the entire body contacting face of the dressing although adhesive free margins may be provided for use as handles if desired).

The adhesive is preferably one which itself transmits water vapour, for example one which if present as a film 25µm thick (which is free of pores whether macroporous or microporous) would have a MVP of at least 300g/m², more suitably at least 500g/m² and preferable at least 700g/m². Such permeabilities are enhanced by using a porous adhesive. Suitable adhesives include polyvinyl ethyl ether adhesive and acrylate surgical adhesives.

Preferred acrylate surgical adhesives include those described in European Patent Application No. 81300847 (Publication No. 0035399).

Another preferred acrylate surgical adhesive is the acrylic emulsion polymer known as Primal N 580 (Trade mark, available from Rohm and Haas Co.). Primal N 580 is a 100% acrylic emulsion polymer having a solids content of between 54.5 and 55.5%. The polymer contains carboxyl groups which may be neutralised with, for example, ammonia solution to thicken the liquid emulsion prior to spreading on a release paper. To form a porous adhesive layer this emulsion is mixed with a petroleum ether and ammonia solution, spread on a release paper and the solvents removed. Other acrylate adhesives containing carboxyl groups may be similarly applied.

The dressing of the invention may be made by any convenient process, for example a film of, for example hydrophilic, polyether polyurethane may be coated with a solution or suspension of the adhesive in a volatile component and rapidly heating to produce the porous layer which is then normally covered with a protector. The method of British Patent Specification No. 1563695 may be employed with advantage. The coated films may then be cut, packaged and sterilised in conventional manner, for example by irradiation, heat or ethylene oxide.

Alternatively, the adhesive layer may be formed in a porous form by casting an emulsion of the adhesive onto a release paper, allowing to stand for a short period and then removing the solvent by passage through a drying oven. This porous adhesive layer may then be transfer coated to the film by passage between two rollers set preferably at their lowest nip pressure and at room temperature to avoid the risk of damaging the pores formed in the adhesive.

The area of the pores in the adhesive layer may be varied by varying the ratio of water to lower boiling solvent in the emulsion and the drying temperature and the amount of time after spreading before entering the drying oven. In general the more important parameter is the length of time after spreading and before drying which is generally between 30 seconds and 2.5 minutes, more aptly between 45 seconds and 1.5 minutes, for example about 1 minute.

In a favoured aspect this invention provides a dressing as hereinbefore described in sterile form. Most aptly the sterile dressing is packaged in a bacterial-proof package such as paper or aluminium foil pouch.

Suitable polyurethanes may be produced by the methods of British Patent Specification No. 2093190A on page 6 line 35 to page 8 line 41 thereof.

Normally the dressings are provided for use with silicone release paper (or other convenient material) to protect the adhesive. This protector is removed prior to use of the dressing. The dressings may also be provided with a support layer over the film if required which support layer is removed on the application of the dressing. Naturally neither protector of support layer are essential features of the dressing since neither perform any function when the dressing is in use.

The dressing according to this invention is provided in sterilised form and it is self adhesive and is adhered to a sterile, removable protector layer and packaged in a bacteria-proof package such as a paper, plastics or aluminum foil pouch. Sterilisation may be achieved in conventional manner, e.g. by use of gamma irradiation, heat or ethylene oxide. Suitable forms of removable protectors and support layers for use with the dressing include ones similar to those described in European Patent Applications Nos. 51935, 81987 and 81989 and United Kingdom Application No. 2120104 and United Kingdom Patent No. 1280631.

The dressings of the present invention will be particularly suitable for use in treating wounds which produce large volumes of exudate and also for use as an intravenous dressing (usually abbreviated to I.V. dressing) for securing an indwelling catheter or cannula to reduce the risk of infection at the injection site. The dressings of the present invention are particularly suitable in providing a bacteria-proof adhesive dressing which when placed on healthy skin has a sufficient moisture vapour permeability to prevent its maceration. When used on wounds such as ulcers or donor sites which produce large volumes of exudate the dressings of the invention aid in reducing the amount of exudate retained under the dressing while also aiding in preventing the wound from drying out if it ceases to produce large amounts of exudate. This aids in the healing of the wound.

The following Examples illustrate the invention:

### Demonstration 1

### "Dry" MVP Determination

Discs of the material under test are clamped over Payne Permeability Cups (flanged metal cups) using sealing rings and screw clamps. The exposed surface area of the test sample is 10cm². Each cup contains aproximately 10ml. of distilled water.

After weighing the cups are placed in a fan assisted electric over which is maintaine at 37±° C. The relative humidity within the oven is maintained at approximately 10% by placing 1kg. of anhydrous 3-8 mesh calcium chloride on the floor of the oven.

The cups are removed after 24 hours, allowed to cool for 20 minutes and re-weighed. The MVP of the test material is calculated from the weight loss and expresed in units of grams of weight per square metre per 24 hours.

### "Wet" MVP determination

The method described above is employed except that the Payne Cups are inverted in the oven so that the water within the cups is in contact with the test material.

### Demonstration 2

A solution of hydrophilic polyurethane (of Example 2 of GB No. 2093190A) in industrial methylated spirits (18% soids) was cast using a doctor blade onto a silicone treated release paper (Steralease 50) (Trade mark) to produce a coating weight after drying of 30±3g/m². The cast film was dried at 80° C to remove solvent.

### Demonstration 3

Hydrophilic polyurethanes which contain 15%, 45% and 65% by weight of water when hydrated may be prepared using the method described in Example 2 of British Application No. 2093190 using the following starting mixtures:

Hydrophilic polyurethane (15% water content when hydrated) is formed from polyethylene glycol (M.W. 1540, 15.4g, 0.01 mole), polypropylene glycol (M.W. 1025, 123.0g, 0.12 mole), ethane diol (9.3g, 0.15 mole), 4,4'dicyclohexylmethane di-isocyanate (70.0g, 0.28 mole), di-n-butyl tin dilaurate 0.2% w/w.

Hydrophilic polyurethane (45% water content when hydrated) is formed from polyethylene glycol (M.W. 1540, 154.0g, 0.1 mole), ethane diol (18.6g, 0.3 mole), 4,4' dicyclohexyl methane di-isocyanate (100g, 0.4 mole), di-n-butyl tin dilaurate 0.2% w/w.

Hydrophilic polyurethane (65% water content when hydrated) is formed from polyethylene glycol (M.W. 6000, 60g, 0.01 mole), ethane diol (7.44g, 0.12 mole), 4,4'dicyclohexylmethane di-isocyanate (32.5g, 0.13 mole), di-n-butyl tin dilaurate 0.2% w/w.

### Example 1

An emulsion was prepared by high shear mixing of a polyvinyl ether adhesive (ex Union Carbide) (25%), petroleum spirit (40/60) (25%) and water (50%). This emulsion was coated onto a silicone release paper (Steralease 77) using a doctor blade and dried after 1 minute in a tunnel at 85° C to give an adhesive coating of 30g/m². The dried adhesive was laminated to a film of hydrophilic polyurethane (of Demonstration 2) by transfer coating by passage between two roller using the minimum nip pressure between the rollers of about 137 k Pa (20 psi) gauge at room temperature.

The laminate was cut into 10cm x 15cm portions and sealed into bacteria proof packages and sterilised with ethylene oxide.

The dry-MVP of the dressing was approximately 1590g/m² and the wet-MVP of the dressing was approximately 4750g/m².

### Example 2

The procedure of Example 1 was followed except that the adhesive emulsion was composed of Primal N 580 (50/50 water/acrylic polymer emulsion ex Rohm & Haas) (80g) and petroleum spirit (40/60). The dry-MVP of the dressing was approximately 1300g/m² and the wet-MVP of the dressing was approximately 5250g/m².

### Example 3

A porous vinyl ether adhesive layer was applied to the film of Demonstration 2 as follows. The polyurethane film supported on its release paper was coated with an emulsion prepared by high shear mixing of polyvinyl ethyl ether adhesive (33%) (ex BASF), petroleum spirit (40/60) (17%) and water (50%). The coating was achieved using a doctor blade and after 1 minute the emulsion was dried at 85° C in a tunnel to give a coating weight of 30g/m².

The dry-MVP of the dressing was approximately 1800g/m² and the wet-MVP of the dressing was approximately 4550g/m².

### Example 4

The procedure of Example 1 was repeated using 22g/m² of adhesive. The dry-MVP of the dressing was approximately 1650g/m² and the wet-MVP of the dressing was approximately 5100g/m².

### Example 5

An adhesive emulsion was formed from a mixture of a 50/50 water/acrylic polymer emulsion (100 parts, Primal N 580, available from Rohm and Haas), petroleum ether, bp fraction 40-60, (25 parts) and ammonia solution (0.4 parts) by stirring the components together using a high speed mixer for about 2 minutes until the emulsion had thickened. After allowing to stand for one hour the emulsion was spread onto a silicone release paper from a spreading box using a gap of 150 µm and the solvents removed at 80° C. A sample of the resulting adhesive layer was examined in a scanning electron microscope so that the dimension of the pores could be determined. The pores had a diameter from 20µm to 300µm. The weight of the adhesive per unit area was 40g/m². The pores accounted for an area of about 20% of the surface area of the adhesive.

A film of a hydrophilic polyurethane prepared in the manner described in Demonstration 2 was cast to give a film which had a weight per unit area of 25g/m². The porous acrylic adhesive film was then transfer coated onto the polyurethane film. The resulting adhesive coated polyurethane film had a dry-MVP of 1180gm⁻² and a wet-MVP of 3890gm⁻².

### Example 6

A hydrophilic polyurethane was prepared by reacting together a polyethylene glycol (M.W. 1486. 45g), polypropylene glycol (M.W. 1024, 275.5g), 4,4'dicyclohexylmethane di isocyanate (112g) using diaminoethane (18g) as a chain extender. The reaction was carried out in methylene chloride solution. The polyurethane formed contained about 24% water when hydrated. A film was cast from the solution at the end of the reaction, to give a film which had a weight per unit area of 30g/m²

A porous acylic adhesive film prepared as described in Example 5 was transfer coated onto this film. The resulting adhesive coated polyurethane film had a dry-MVP of 1680gm⁻² and a wet-MVP of 3240gm⁻².

### Example 7

An adhesive emulsion was formed from a mixture of a 50/50 water/acrylic polymer emulsion (100 parts, Primal N 580, available from Rohm and Haas), petroleum ether, bp fraction 60-80° C (20 parts), petroleum ether, bp fraction 100-120° C (5 parts) and ammonia solution (0.4 parts) by stirring the components together using a high speed mixer for about 2 minutes until the emulsion thickened. The adhesive emulsion was allowed to stand for 1 hour and then spread onto a siliconised release paper from a spreading box set at a gap of 150µm. The solvents were removed at 80° c. A sample of the resulting adhesive layer was examined under a scanning electron microscope and the dimensions of the pores determined. The majority of the pores were from 50 to 100µm in diameter. The weight of adhesive per unit area was 50g/m² and the pores accounted for about 20% the area of adhesive.

A film of hydrophilic polyurethane prepared in a similar manner to that described in Demonstration 2 was cast to give a film which had a weight per unit area of 20g/m². The porous acrylic adhesive film was then transfer coated onto the polyurethane film. The resulting adhesive-coated polyurethane film had a dry-MVP of 1730gm⁻² and a wet-MVP of 3880gm⁻².

### Example 8

A film of hydrophilic polyurethane of the type disclosed in British Application No. 2093190A and which contained 45% by weight of water when hydrated was cast into a film in a manner similar to that described in Demonstration 2. The film had a weight per unit area of 18g/m² and a thickness of about 20µm.

A porous acrylic adhesive film prepared in the same manner to that of example 7 was transfer coated to the hydrophilic polyurethane. The resulting adhesive coated film had a dry-MVP of 1840gm⁻² and a wet-MVP of 4850gm⁻².

### Example 9

A film of hydrophilic polyurethane of the type disclosed in British Application No. 2093190 and whch contained 65% by weight water when hydrated was cast into a film in a manner similar to that described in Demonstration 2. The film had a weight per unit area of 20g/m² and a thickness of about 22µm.

A porous acrylic adhesive film prepared in the same manner to that of Example 7 was transfer coated to the hydrophilic polyurethane. The resulting adhesive-coated polyurethane film had a dry-MVP of 1960gm⁻² and a wet-MVP of 6060gm⁻².

### Example 10

A film of hydrophilic polyurethane of the type disclosed in British Application No. 2093190A and which contained 15% by weight water when hydrated was cast into a film in a manner similar to that described in Demonstration 2. The film had a weight per unit area of 20g/m² and a thickness of about 20µm.

A porous acrylic adhesive film prepared in a similar manner to that of Example 7 was transfer coated to the hydrophilic polyurethane. The majority of the pores in the adhesive were greater than 100µm in diameter and accounted for approximately 28% of the area of the adhesive. The resulting adhesive coated film had a dry-MVP of 1810gm⁻² and a wet-MVP of 2600gm⁻².

### Example 11

A polyether-polyamide polymer (Pebax 4011 RN00), available from ATO Chemical Products (U.K.) Ltd) was extruded to form a flat film 25µm in thickness. This polymer contains approximately 55% by weight of water when hydrated.

A porous acrylic adhesive film was prepared on a silicone release paper in the manner as that of Example 7 so that the weight of adhesive per unit area was 50g/m², the majority of the pores were from 50 to 100µm in diameter and formed about 21% of the area of the adhesive.

The porous adhesive layer was transfer coated to the polyether-polyamide polymer film by passing the films together between two rollers at a minimun nip pressure of about 137k Pa (20 psi) and at room temperature. The resultant laminate comprising a polyether polyamide-acrylic adhesive-releae paper may be cut up into portions 7.5cm x 7.5cm, sealed into bacteria proof pouches and sterilised with ethylene oxide. In use the dressing is removed from the pouch, the release paper is removed and the dressing is attached to the skin surrounding the wound by means of the adhesive.

The dry-MVP of the dressing was approximately 1910gm⁻² and the wet-MVP was 5180gm⁻².

### Example 12

An adhesive emulsion was prepared from a mixture of an acrylic polymer emulsion (Primal N 580. 100 parts), petroleum ether, bp fraction 40-60, (25 parts) and ammonia solution (0.4 parts) by stirring the components together using a high speed mixer for about 2 minutes until the emulsion had thickened. After allowing to stand for 1 hour the emulsion was spread onto a silicone release paper from a spreading box using a gap of 150µm. The solvents were then removed at 80°C. A sample of the resulting adhesive layer was examined in a scanning electron microscope so that the dimensions of the pores and the area of the adhesive they account for can be determined. The pores had a diameter of 20 to 300µm, with a majority of pores being greater than 100µm. The pores accounted for approximately 25% of the area of the adhesive. The weight of adhesive per unit area was 40g/m².

A film of hydrophilic polyurethane prepared in the manner described in Demonstration 2 gave a film which had a weight per unit area of 25g/m². The porous acrylic adhesive layer was then transfer coated onto the polyurethane film by passing the respective film and layers on their release papers between a pair of rollers at room temperature. The release paper was removed from the adhesive layer and replaced by a split protector which covered the whole of the adhesive.

The laminate strip so formed comprising hydrophilic polyurethane-adhesive-protector may be cut to give dressings 7.5 x 7.5cm. The individual dressings may be placed and sealed in a bacteria proof pack and sterilised.

In use the sterile dressing is removed from the pack, the two parts of the protector pulled back a little way to expose a small area of adhesive which may be accurately and smoothly placed where required. The remainder of the adhesive surface is then exposed and by complete removal of the two parts of the protector.

### Example 13

A porous acrylic adhesive layer was formed on a silicone release paper in a manner similar to that described in Example 12.

A film of hydrophilic polyurethane prepared in the manner described in Demonstration 2 gave a film with a weight per unit area of 25g/m². The porous acrylic adhesive layer was then transfer coated onto the polyurethane film by passing the film and adhesive layer on their release papers between a pair of roller at room temperature. The laminate so formed may be cut in such a way as to provide the dressings having the structure shown in Figures 1 and 2 of European Patent Application No. 51939, so that the hydrophilic polyurethane has a removable support layer either all over its non-adhesive surface or a support layer in the form of a removable frame.

The individual dressings may be placed and sealed in a bacteria proof pack and sterilised.

## Claims

1. A surgical dressing which consists essentially of a film which carries over its face an adhesive layer for securing the dressing to the body characterised in that (a) the film is continuous, hydrophilic and elastomeric and consists essentially of polyether polyurethane, polyether polyamide block copolymer or polyether polyester block copolymer and when in contact with water has a higher MVP than when in contact with moisture vapour but not water (b) the adhesive layer is porous, allows access of water to the film when water is in contact with the adhesive layer and is applied over the whole of the operative area of the surgical dressing so that (c) said surgical dressing has a wet-MVP of not less than 2500g/m² when the adhesive layer is in contact with water and a dry-MVP of not more than 2000g/m² when the adhesive is in contact with moisture vapour but not water, whereby the dressing is suitable for use on exuding wounds and on non-exuding wounds, the pores in said adhesive being from 20 to 300µm in diameter and accounting for 10 to 50% of the area of the adhesive.

2. A dressing as claimed in claim 1 wherein the dry-MVP is not more than 1500g/m² when the adhesive layer is in contact with moisture vapour but not water.

3. A dressing as claimed in either of claims 1 or 2 wherein the wet-MVP is not less than 3200g/m² when the adhesive layer is in contact with water.

4. A dressing as claimed in any of claims 1 to 3 wherein the film comprises a hydrophilic polyether polyurethane which when hydrated contains 5% to 50% of water and is from 15 to 80µm thick.

5. A dressing as claimed in claim 4 wherein the film comprises a hydrophilic polyether polyurethane which when hydrated contains 10% to 40% of water and is from 20 to 60µm thick.

6. A dressing as claimed in any of claims 1 to 3 wherein the film is a hydrophilic polyether polyurethane.

7. A dressing as claimed in any of claims 1 to 6 wherein the adhesive compries a polyvinyl ethyl ether or an acylate surgical adhesive.

8. A dressing as claimed in any of claims 1 to 7 in which the average weight per unit area of adhesive is 20g/m² to 45g/m².

9. A dressing as claimed in any of claims 1 to 8 in sterile form packaged in a bacteria proof package.

## Patentansprüche

1. Chirurgisches Verbandmittel, welches im wesentlichen aus einem Film besteht, welcher eine Klebeschicht über seiner Oberseite zum Befestigen des Verbandmittels am Körper trägt, dadurch gekennzeichnet, daß (a) der Film kontinuierlich, hydrophil und elastomer ist und im wesentlichen aus Polyetherpolyurethan, Polyetherpolyamid-Blockcopolymer oder Polyetherpolyester-Blockcopolymer besteht und wenn er sich in Kontakt mit Wasser befindet, eine höhere Wasserdampfdurchlässigkeit besitzt, als wenn er sich in Kontakt mit Feuchtigkeitsdampf, nicht aber Wasser befindet, (b) die Klebeschicht porös ist, den Zutritt von Wasser zu dem Film gestattet, wenn sich Wasser in Kontakt mit der Klebeschicht befindet und über die gesamte wirksame Fläche des chirurgischen Verbandmittels dergestalt aufgebracht ist, daß (c) genanntes chirurgisches Verbandmittel eine Naßwasserdampfdurchlässigkeit von nicht weniger als 2500 g/m² besitzt, wenn sich die Klebeschicht in Kontakt mit Wasser befindet und eine Trockenwasserdampfdurchlässigkeit von nicht mehr als 2000 g/m² besitzt, wenn der Kleber sich in Kontakt mit Feuchtigkeitsdampf, nicht aber Wasser befindet, wobei das Verbandmittel zur Verwendung auf nässenden Wunden und auf nicht nässenden Wunden geeignet ist, wobei die Poren in genannten Kleber von 20 bis 300 um im Durchmesser sind und 10 bis 50% der Fläche des Klebers ausmachen.

2. Verbandmittel nach Anspruch 1, worin die Trockenwasserdampfdurchlässigkeit nicht mehr als 1500 g/m² beträgt, wenn sich die Klebeschicht in Kontakt mit Feuchtigkeitsdampf, nicht aber mit Wasser befindet.

3. Verbandmittel nach einem der Ansprüche 1 oder 2, worin die Naßwasserdampfdurchlässigkeit nicht weniger als 3200 g/m² beträgt, wenn sich die Klebeschicht in Kontakt mit Wasser befindet.

4. Verbandmittel nach einem der Ansprüche 1 bis 3, worin der Film ein hydrophiles Polyetherpolyurethan umfaßt, welches, wenn hydratisiert, 5 bis 50% Wasser enthält und von 15 bis 80 µm dick ist.

5. Verbandmittel nach Anspruch 4, worin der Film ein hydrophiles Polyetherpolyurethan umfaßt, welches, wenn hydratisiert, 10 bis 40% Wasser enthält und von 20 bis 60 µm dick ist.

6. Verbandmittel nach einem der Ansprüche 1 bis 3, worin der Film ein hydrophiles Polyetherpolyurethan ist.

7. Verbandmittel nach einem der Ansprüche 1 bis 6, worin der Kleber einen Polyvinylethylether oder einen chirurgischen Akrylatkleber umfaßt.

8. Verbandmittel nach einem der Ansprüche 1 bis 7, worin das durchschnittliche Gewicht pro Flächeneinheit Kleber 20 g/m² bis 45 g/m² beträgt.

9. Verbandmittel nach einem der Ansprüche 1 bis 8 in steriler Form in einer bakteriendichten Packung verpackt.

## Revendications

1. Pansement chirurgical qui consiste essentiellement en une couche mince qui porte sur sa face une couche adhésive, pour fixer le pansement au corps, caractérisé en ce que (a) la couche mince est continue, hydrophile ayant la qualité de l'élastomère et consiste essentiellement en polyuréthane de polyéther, a copolymère groupé de polyamide de polyéther ou en copolymère groupé de polyester de polyéther et lorsqu'elle est en contact avec de l'eau, a une plus grande perméabilité à la vapeur humide (PVH) que lorsqu'elle est en contact avec de la vapeur humide mais pas avec de l'eau, (b) la couche adhésive est poreuse, permet à l'eau d'accéder à la couche mince quand l'eau est en contact avec la couche adhésive, et elle est appliquée sur toute la surface active du pansement chirurgical, de sorte que (c) ledit pansement chirurgical a une PVH au mouillé qui n'est pas inférieure à 2500 g/m² quand la couche adhésive est en contact avec l'eau et a une PVH à sec qui n'excède pas 2000 g/m² quand l'adhésif est en contact avec de la vapeur humide mais pas avec de l'eau, grâce à quoi le pansement convient pour une utilisation sur des plaies exsudantes et sur des plaies non-exsudantes, les pores dudit adhésif faisant de 20 à 300 µm de diamètre et représentant de 10 à 50% de la surface de l'adhésif

2. Pansement selon la revendication 1, dans lequel la PVH à sec n'excède pas 1500 g/m² quand la couche adhésive est en contact avec de la vapeur humide mais pas avec de l'eau.

3. Pansement selon la revendication 1 ou 2, dans lequel la PVH au mouillé n'est pas inférieure à 3200 g/m² quand la couche adhésive est en contact avec de l'eau.

4. Pansement selon l'une quelconque des revendications 1 à 3, dans lequel la couche mince comprend un polyuréthane de polyéther hydrophile qui contient 5 à 50% d'eau lorsqu'il est hydraté et elle a une épaisseur de 15 à 80 µm.

5. Pansement selon la revendication 4, dans lequel la couche mince comprend un polyuréthane de polyéther hydrophile qui contient 10 à 40% d'eau lorsqu'il est hydraté et elle a une épaisseur de 20 à 60 µm.

6. Pansement selon l'une quelconque des revendications 1 à 3, dans lequel la couche mince est un polyuréthane de polyéther hydrophile.

7. Pansement selon l'une quelconque des revendications 1 à 6, dans lequel l'adhésif comprend un adhésif chirurgical à base d'éther éthylique de polyvinyle ou à base d'acrylate.

8. Pansement selon l'une quelconque des revendications 1 à 7, dans lequel le poids moyen par unité de surface d'adhésif est compris entre 20 g/m² et 45 g/m².

9. Pansement selon l'une quelconque des revendications 1 à 8, conditionné sous forme aseptique dans un emballage résistant aux bactéries.
